# EUROPEAN PATENT SPECIFICATION

(11) **EP 3 387 143 B1**
(45) Date of publication and mention of the grant of the patent: **18.09.2019**
(21) Application number: 16808742.7
(22) Date of filing: 28.11.2016
(51) Int. Cl.: C12Q 1/04, C12Q 1/18, C12Q 1/34

(54) **EXTRACTION DISC**
EXTRAKTIONSSCHEIBE
DISQUE D'EXTRACTION

(30) Priority: 11.12.2015 GB 201521899
(43) Date of publication of application: 17.10.2018
(73) Proprietor: Mast Group Limited, Liverpool, Merseyside L20 1EA (GB)
(72) Inventor: BLUNDELL, Jamie, Liverpool Merseyside L24 5RY (GB); HOBSON, Jonathan Anthony, Liverpool Merseyside L25 3PE (GB)
(74) Representative: Rees, Kerry
(86) International application number: PCT/GB2016/053732
(87) International publication number: WO 2017/098206

(56) References cited:
- WO-A1-2009/051838
- US-A1- 2007 190 592
- A. J. MATHERS ET AL: "Modified Hodge Test versus Indirect Carbapenemase Test: Prospective Evaluation of a Phenotypic Assay for Detection of Klebsiella pneumoniae Carbapenemase (KPC) in Enterobacteriaceae", JOURNAL OF CLINICAL MICROBIOLOGY, vol. 51, no. 4, 1 April 2013 (2013-04-01), pages 1291-1293, XP055347527, US ISSN: 0095-1137, DOI: 10.1128/JCM.03240-12 cited in the application
- J. A. BLACK ET AL: "AmpC Disk Test for Detection of Plasmid-Mediated AmpC -Lactamases in Enterobacteriaceae Lacking Chromosomal AmpC -Lactamases", JOURNAL OF CLINICAL MICROBIOLOGY, vol. 43, no. 7, 1 July 2005 (2005-07-01), pages 3110-3113, XP055347519, US ISSN: 0095-1137, DOI: 10.1128/JCM.43.7.3110-3113.2005 cited in the application
- MOLAND E S ET AL: "Newer beta-Lactamases: Clinical and Laboratory Implications, Part II", CLINICAL MICROBIOLOGY NEWSLETTER, ELSEVIER, NEW YORK, NY, US, vol. 30, no. 11, 1 June 2008 (2008-06-01), pages 79-85, XP022672858, ISSN: 0196-4399, DOI: 10.1016/J.CLINMICNEWS.2008.05.001 [retrieved on 2008-05-22]
- PETER S ET AL: "Evaluation of phenotypic detection methods for metallo-[beta]-lactamases (MBLs) in clinical isolates ofPseudomonas aerugi", EUROPEAN JOURNAL OF CLINICAL MICROBIOLOGY & INFECTIOUS DISEASES, SPRINGER, WIESBADEN, DE, vol. 33, no. 7, 23 January 2014 (2014-01-23), pages 1133-1141, XP035313744, ISSN: 0934-9723, DOI: 10.1007/S10096-014-2059-1 [retrieved on 2014-01-23]

## Description

### FIELD OF INVENTION

The present invention relates to a method for detecting the presence of a carbapenem or penem hydrolysing β-lactamase-producing bacteria in a sample, and enzyme assay and kits for use in such methods.

### BACKGROUND ART

Disc diffusion methods used for antibiotic susceptibility testing, including the Modified Hodge-Test, are widely used to determine what antibiotics are likely to be effective against a disease causing bacteria since it is common knowledge that certain bacterial enzymes can inactivate particular antibiotics thereby rendering treatment with them ineffective. Variations of disc based diffusion methods, such as the MHT, can be used to demonstrate production of antibiotic inactivated enzymes.

Generally in such disc diffusion methods the infectious microorganism is uniformly spread over the surface of a culture medium, such as an agar plate, then several filter paper discs impregnated with specific amounts of selected antibiotics are placed on the agar surface. During incubation, the microorganism grows on the surface of the agar except in the areas where certain antibiotics have inhibited its growth. Inhibition of growth is detected as clear zones of no growth (inhibition zones) on the agar around the specific antibiotic disc.

Carbapenemases are a group of β-lactamases that are active against various antibiotics including carbapenems. Carbapenemases belong to three classes of beta-lactamases, namely Ambler class A, Ambler class B and Ambler class D. These three classes of carbapenemases confer resistance in clinically important bacteria to carbapenems or decreased susceptibility to carbapenems. Bacteria that produce carbapenemases are therefore of interest clinically and methods for their early detection are required in order to prevent their spread and to reduce multidrug and pandrug resistance.

Two tests are widely used clinically to detect carbapenemases producing bacteria; the "Etest®" and other MIC strip products and the "Modified Hodge-Test". In the "Etest®" antimicrobial agents are tested against a test microorganism provided in opposing, predefined gradients of indicator carbapenem and carbapenem plus enzyme inhibitor on agar. This is used to determine the Minimum Inhibitory Concentration (MIC) of the antimicrobial agent. In the "Modified Hodge-Test" carbapenemase producing bacteria are detected when a reporter bacterium grows toward a carbapenem containing disc to produce a characteristic "clover-leaf' growth pattern around the disc. This phenomenon can be mediated by carbapenem inactivating enzymes produced by the test isolate

Black J. et al (Journal of Clinical Microbiology, July 2005, p. 3110-3123) reported an AmpC disc test, based on filter paper discs impregnated with EDTA to detect plasmid-mediated AmpC B-lactamase in *Enterobacteriaceae* lacking a chromosomally mediated AmpC B-lactamase using cefoxitin insusceptibility as a screen.

Pasteran F. et al (Journal of Clinical Microbiology, December 2011, p. 4301-4303) discloses a Modified Hodge Test for the detection of *Klebsiella pneumoniae* carbapenemase (KPC) and metallo-Beta-Lactamase (MBL) expression in *P. Aeruginosa* using a *Klebsiella pneumoniae* indicator.

Mathers. A et al (Journal of Clinical Microbiology, April 2013, p. 1291-1293) discloses an indirect carbapenemase test (ICT) for the detection of *K. pneumoniae* carbapenemase production in *Enterobacteriaceae* and, specifically, in *Enterobacter* spp.. *Klebsiella* spp.. *Citrobacter freundii and E.coli.* Briefly, ICT involves placing an EDTA disc which is coated with 2-3 colonies of the test isolate adjacent to a disc containing imipenem (a carbapenem antibiotic) on a lawn of *E.coli* and evaluating the growth of *E.coli* in a zone of inhibition.

There is significant difficulty associated with the detection of carbapenemase producing *Enterobacteriaceae* (CPE), when performing first line panel testing in hospital laboratories, due to the diverse susceptibility patterns that vary between different types of carbapenemase enzymes (MBL, OXA, and KPC). It is often found that Modified Hodge Tests and ICTs are not always reliable for the detection of CPE and they fail to detect a substantial proportion of CPE isolates or misidentify isolates producing ESBL or AmpC β-lactamases, particularly when those isolates are also porin deficient. The present invention addresses this and other problems associated with the prior art methods.

### SUMMARY OF THE INVENTION

The inventors have developed a method that is effective at identifying bacteria which produce β-lactamses, particularly KPCs, MBLs and OXA-48s. The new test is particularly effective at identifying MBL production in *Enterobacteriaceae, Pseudomonas* spp or *Acinetobacter* spp.

Thus, in a first aspect the present invention provides a method for determining whether a microorganism produces a carbapenem or penem hydrolysing β-lactamase, comprising:
providing a reagent composition comprising a β-lactam containing antibiotic, an Extended-Spectrum β-lactamase inhibitor or an AmpC inhibitor to which any non-carbapenem β-lactamase in the sample is susceptible, and a membrane permeabilizing agent which is ethylene glycol tetraacetic acid (EGTA) that permeabilizes the microorganism's outer membrane;
contacting the microorganism suspected of producing a carbapenem β-lactamase with the reagent composition; and
culturing the microorganism with the reagent composition in a culture medium comprising a reporter microorganism wherein a positive test is determined by an increase in the growth of the reporter microorganism.

Preferable, the reagent composition is provided on an enzyme assay disc comprising a solid support on which the reagent composition is impregnated or absorbed.

Optionally, each of the components of the reagent composition may be impregnated or absorbed on a single assay disc. Alternatively, a β-lactam containing antibiotic may be impregnated or absorbed on an assay disc, with an Extended-Spectrum β-lactamase inhibitor or an AmpC inhibitor and EGTA impregnated or absorbed on one or more additional assay discs.

Optionally, a negative control disc may also be provided. The negative control disc may be impregnated with an Extended-Spectrum β-lactamase inhibitor or an AmpC inhibitor and EGTA. The negative control disc may additionally be impregnated with a negative control organism. Suitable organisms may be carbapenemase sensitive, such as *Escherichia coli* ATCC25922.

Optionally, the negative control disc may also comprise a β-lactam containing antibiotic.

In a second aspect the present invention provides an enzyme assay system for use in a method of determining whether a microorganism produces a carbapenem or penem-hydrolysing β-lactamase, the system comprising: a β-lactam containing antibiotic; an extended-spectrum β-lactamase inhibitor and/or an AmpC inhibitor to which the carbapenem or penem hydrolysing β-lactamase is resistant; and a membrane permeabilizing agent which is ethylene glycol tetraacetic acid (EGTA) that permeabilizes the microorganism's outer membrane; wherein the system has one or more assay discs and the a β-lactam containing antibiotic, extended-spectrum β-lactamase inhibitor and/or an AmpC inhibitor, and ethylene glycol tetraacetic acid (EGTA) are impregnated in the one or more discs.

In a third aspect the present invention provides a kit for determining whether a microorganism produces a carbapene- hydrolysing β-lactamase, comprising an enzyme assay system according to the second aspect of the present invention.

### DETAILED DESCRIPTION OF THE INVENTION

The method of the invention may be used to determine whether a microorganism produces a carbapenem or penem-hydrolysing β-lactamase. Preferably, the β-lactamases that may be detected are selected from metallo-β-lactamases (MBL), *Klebsiella pneumoniae* carbapenemase (KPC) and Oxacillinases e.g. OXA-48 carbapenemase.

Preferably, the β-lactamase is produced by a *Pseudomonas spp.,* Enterobacteriaceae such as *E. coli, Klebsiella spp. Citrobacter freundii* and/or *Enterobacter spp.*

Carbapenemases are clinically important because they destroy, and so may confer resistance to, carbapenems, and usually most other β-lactams, including penem antibiotics. The five main carbapenemase families found in the UK, the so-called 'big five', consist of IMP, VIM and NDM metallo-β-lactamases capable of hydrolyzing all β-lactam antibiotics except monobactams, KPC with activity against all β-lactams and OXA enzymes which may retain susceptibility only to broad-spectrum cephalosporin within the β-lactam class of antibiotics.

The method of the present invention is particularly advantageous in the detection of MBL. Preferably the MBL is produced by *Pseudomonas* spp. MBL requires a divalent cation, such as zinc, to function. The prior art methods employ EDTA either on its own or paired with one antibiotic to eliminate false positive results. However, EDTA, as well as acting as a permeabilization agent, acts to chelate zinc ions thereby potentially leading to false negative results. The method of the present invention uses ethylene glycol tetraacetic acid (EGTA) which preferentially sequesters calcium ions as opposed to zinc ions which reduces the chances of obtaining a false negative result, This allows positive results to be observed in the method of the present invention for the presence of MBL enzymes. Thus the reagent composition used in the present invention a β-lactam containing antibiotic, an extended-spectrum β-lactamase inhibitor and/or an AmpC inhibitor to which the carbapenem hydrolysing β-lactamase is resistant, and a membrane permeabilizing agent which is ethylene glycol tetraacetic acid (EGTA) that permeabilizes the microorganism's outer membrane. The addition of zinc to the reagent composition used in the method of the present invention prevents false negatives. The addition of zinc is not possible in the prior art methods where EDTA is used. Thus the reagent composition may contain a divalent cation which is preferably zinc ions.

The β-lactam containing antibiotic used in the reagent composition may be selected from carbapenem or penem, preferably selected from meropenem, imipenem, ertapenem, doripenem, faropenem, sulopenem, ritapenem.

In order to prevent false positive results from non-carbapenem β-lactamases that may be produced by the test microorganism, an extended spectrum β-lactamase inhibitor and/or an AmpC inhibitor to which the carbapenem β-lactamase which is produced by the test microorganism is resistant may be present in the reagent composition. Preferably, the inhibitors are selected from cloxacillin and tazobactam particularly when the organism is suspected of producing an enzyme which confers carbapenem resistance, Other inhibitors include flucloxacillin, oxacillin, nafcillin, dicloxacillin, clavulanic acid and sulbactam.

The reagent composition used in the invention comprises a β-lactam containing antibiotic. Preferably the β-lactam containing antibiotic is selected from faropenem, meropenem, ertapenem and doripenem or mixtures thereof. Preferably the antibiotic is faropenem.

The test microorganism is cultured with the reagent composition in a culture medium. The culture medium is any culture medium suitable for culturing a given microorganism which would be known to the skilled person. Preferably, the culture may be a liquid or a solid culture medium. Preferably the culture medium is a solid culture medium such as Mueller Hinton agar.

In one aspect of the invention the reagent composition is provided in at least two separate discs wherein each disc comprises a carrier and the first disc has the β-lactam containing antibiotic impregnated in its carrier and the second disc has an extended-spectrum β-lactamase inhibitor and/or AmpC inhibitor and EGTA impregnated in its carrier.

Typically, each disc is a standard antibiotic susceptibility testing disc having comprising white paper with 290gsm ± 30gsm weight or 140gsm ± 30gsm weight and has a 6mm diameter or 8mm diameter. Alternatively, the disc may have any suitable diameter. Optionally, the disc may have an 8mm diameter.

A reporter microorganism is provided in the culture medium to assess whether the test microorganism produces a carbapenem-hydrolysing β-lactamase. In one aspect, a positive test for a carbapenem-hydrolysing β-lactamase producing microorganism is determined by an increase in the growth of the reporter microorganism. The growth of the reporter microorganism may be observed by the naked eye as a lawn of growth of the organism on a solid growth medium. Preferably, the test microorganism is *E.coli* ATCC25922. Alternatively, the reporter organism is *Klebsiella pneumoniae* ATCC700603. *Klebsiella pneumoniae* is particularly effective for the detection of KPC and MBL expression in *P. Aeurginosa.*

In a preferred embodiment the reagent composition is provided in at least two separate discs wherein each disc comprises a carrier and the first disc has the β-lactam containing antibiotic impregnated in its carrier and the second disc has the extended-spectrum β-lactamase inhibitor and/or AmpC inhibitor and EGTA impregnated in its carrier. The test microorganism is applied to the disc containing the EGTA and the two discs are provided on the solid growth medium. After a sufficient incubation time a positive result is observed where the EGTA porinates the test bacterium to release the carbapenem-hydrolysing β-lactamase enzyme which dissipates in to the surrounding culture medium and provides localized protection to a susceptible reporter microorganism. Thus a positive result is observed by the characteristic distortion of zone of inhibition around the discs containing the test microorganism.

Preferably, the disc having the β-lactam containing antibiotic impregnated in its carrier is obtainable from Mast Group Limited as is designated a D71C disc. Preferably, the β-lactam antibiotic is faropenem because this antibiotic provides a zone of diameter of 18-20 mm when *E.co*/*i* ATCC25922 is used as the reporter microorganism. This result means that when the method of the invention is conducted on a dual disc test system no spacing is needed between the antibiotic disc and the enzyme extraction disc. This is preferable as the antibiotic disc and the enzyme extraction disc may be abutted together. In embodiments where other β-lactam containing antibiotics are used, such as meropenem, a gap may be required between each disc. The gap between the discs may be about 9mm or less. Preferably the gap may be about 3mm.

In one aspect of the invention, the test microorganism is cultured with the reagent composition in a culture medium for from 16 to 24 hours at from 35 to 37°C. Preferably, the β-lactamase inhibitor is Cloxacillin present in an amount of from 67 to 150µg/ disc or Tazobactam from 6 to 15µg/disc or mixtures thereof. Preferably the β-lactam containing antibiotic is present in an amount of from 1-25µg/disc. Preferably, the EGTA is present in an amount of from 150 to 500µg/disc. Preferably, zinc ions are present in the second disc at 75-150µg/disc.

The method of the present invention is able to produce accurate results within 18 to 20 hours after start of incubation of the test microorganism with the reagent composition.

Optionally a negative control disc may further be provided. The negative control disc may be a standard antibiotic susceptibility testing disc. The negative control disc may comprise an extended-spectrum β-lactamase inhibitor and/or AmpC inhibitor and EGTA impregnated in its carrier. The negative control disc may further comprise a control organism which is carbapenemase sensitive, for example *Escherichia coli* ATCC25922. Such discs may need to be positioned at the same or at a similar distance from a β-lactam containing antibiotic contain disc as an extended-spectrum β-lactamase inhibitor and/or AmpC inhibitor and EGTA impregnated extraction disc in order that the control disc be in the same environment as the extraction disc. Alternatively, the negative control disc may further comprise a β-lactam containing antibiotic. Such control discs may not have to be positioned at any set point relative to the assay discs used in the method of the present invention.

In one aspect of the invention the reagent composition may be provided as an assay system having three discs which are connected such that the discs are arranged so as to have one central disc (antibiotic disc) and two peripheral discs (extraction and control discs) wherein the relative spacing between the discs is fixed. The central disc may have each of the features of the first disc as set out above, while the two peripheral discs may have the same features and reagent composition as the second disc and/or negative control disc as set out above. The test microorganism is applied to the non-control disc containing the EGTA and the device is provided on the solid growth medium. Preferably the device is placed onto the reporter organism suspension lawn such that the extraction and control tabs make contact with the culture medium. After a sufficient incubation time a positive result is observed where the EGTA porinates the test bacterium to release the carbapenem-hydrolysing β-lactamase enzyme which dissipates in to the surrounding culture medium and provides localized protection to a susceptible reporter microorganism. Thus a positive result is observed by the characteristic distortion of zone of inhibition around the discs containing the presence of dis-similar zones of inhibition between the central antibiotic disc and the peripheral extraction disc containing the test microorganism compared to that of the central antibiotic disc and the negative control disc indicates a positive test for a carbapenem hydrolysing β-lactamase enzyme.

No difference between the zones of inhibition between the central antibiotic disc and the peripheral extraction disc containing the test microorganism compared to that of the central antibiotic disc and the negative control disc indicates a negative test for a carbapenem hydrolysing β-lactamase enzyme.

Optionally, the triple disc assay system may be provided additional support structure. The additional support structure may fix the three discs at optimal positions relative to each other. The additional support structure may also provide easier handling of the system when placing on to a test plate such as a pre-swabbed reporter organism agar plate.

Preferably, the device having three connected discs may be obtainable from Mast Group Limited, designated as a D74 disc. Preferably, the β-lactam antibiotic is cloxacillin. Cloxacillin may be present in an amount from about 67µg to about 150µg. Alternatively, the β-lactam antibiotic is Tazobactam. Tazobactum may be present in an amount from about 6µg to about 25µg. Preferably the β-lactam containing antibiotic is present in an amount of from 1-25µg/disc. Preferably, the EGTA is present in an amount of from 150 to 500µg/disc. Preferably, zinc ions are present at 75-150µg/disc. The device may be made from paper. Optionally, the paper may be white paper with 100 to 320gsm basis weight. Preferably the paper may have 110gsm ± 30gsm basis weight or 290gsm ± 30gsm basis weight.

When the method of the invention is conducted on a triple disc test system the spacing between the discs may be pre-set an optimised for the antibiotic disc and the enzyme extraction disc used. The gap between neighboring discs may be about 9mm or less. Preferably the gap may be about 3mm. The gap between neighboring discs may be small enough such that the zone of inhibition around the negative control disc meets that of the central antibiotic disc.

In another aspect of the invention the reagent composition is provided on a single disc comprising the β-lactam containing antibiotic, the extended-spectrum β-lactamase inhibitor and/or AmpC inhibitor and the EGTA.

The disc is a standard antibiotic susceptibility testing disc having comprising white paper with 290gsm ± 30gsm or 140gsm ± weight and has a 6mm diameter or 8mm diameter. Alternatively, the disc may have any suitable diameter. Optionally, the disc may have an 8mm diameter.

A reporter microorganism is provided in the culture medium to assess whether the test microorganism produces a carbapenem-hydrolysing β-lactamase. In one aspect, a positive test for a carbapenem-hydrolysing β-lactamase producing microorganism is determined by an increase in the growth of the reporter microorganism. The growth of the reporter microorganism may be observed by the naked eye as a lawn of growth of the organism on a solid growth medium. Preferably, the test microorganism is *Klebsiella pneumoniae* ATCC700603. Alternatively, the test microorganism is *E.coli* ATCC25922.

In a preferred embodiment the reagent composition is provided in a single disc wherein the disc comprises a carrier comprising the β-lactam containing antibiotic, the extended-spectrum β-lactamase inhibitor and/or AmpC inhibitor and EGTA impregnated in the carrier. The test microorganism is applied to the disc and the disc is provided on the solid growth medium. After a sufficient incubation time a positive result is observed where the EGTA porinates the test bacterium to release the carbapenem-hydrolysing β-lactamase enzyme which dissipates in to the surrounding culture medium and provides localized protection to a susceptible reporter microorganism. Thus a positive result is observed by the characteristic distortion of zone of inhibition around the disc.

Preferably, the β-lactam antibiotic is faropenem because this antibiotic provides a zone of diameter of 18-20 mm when *E.coli* ATCC25922 is used as the reporter microorganism.

In one aspect of the invention, the test microorganism is cultured with the reagent composition in a culture medium for from 16 to 24 hours at from 35 to 37°C. Preferably, the β-lactamase inhibitor is Cloxacillin present in an amount of from 67 to 150µg/ disc or Tazobactam from 6 to 15µg/disc or mixtures thereof. Preferably the β-lactam containing antibiotic is present in an amount of from 1-25µg/disc. Preferably, the EGTA is present in an amount of from 150 to 500µg/disc. Preferably, zinc ions are present in the second disc at 75-150µg/disc.

The method of the present invention is able to produce accurate results within 16 to 20 hours or 18 to 20 hours after start of incubation of the test microorganism with the reagent composition.

### Figures

Figures 1 and 2 show schematically negative (figure 1) and positive (figure 2) results obtained in the method of the present invention.
Figures 3 shows the results obtained in testing known carbapenem-hydrolysing β-lactamase bacteria in the two disc system of the present invention.
Figure 4 shows a triple disc test system according to an aspect of the present invention.
Figure 5 shows a pictorial representation of the methodology of the set up for using a triple disc test system according to figure 4.

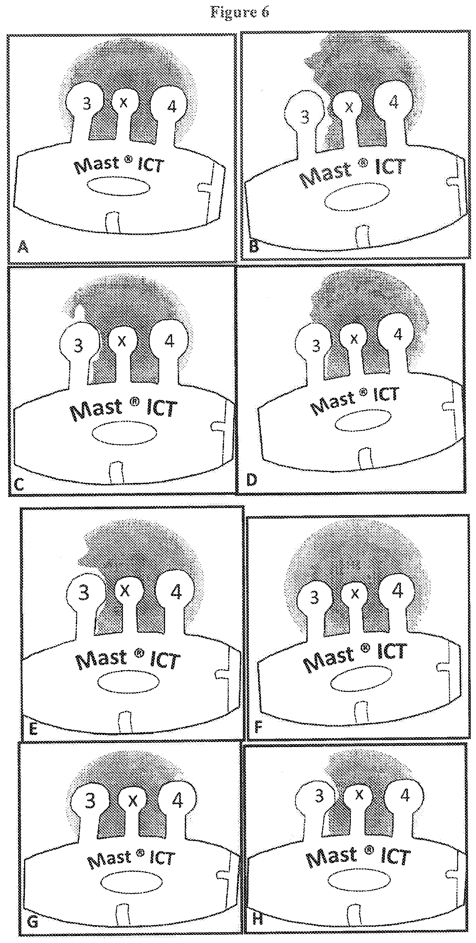

Figure 6 shows the results obtained in testing known carbapenem-hydrolysing β-lactamase bacteria in the three disc system of the present invention: A: Negative test: Carbapenemase negative test organism *E.coli* ATCC®25922 (Tab 3) and *E.coli* ATCC®25922 (Tab 4); B: MBL Positive test: Carbapenemase negative control organism *E.coli* ATCC®25922 (Tab 4) and carbapenemase positive (MBL) test organism *K.Pneumoniae* NCTC 13440 (Tab 3); C: OXA-48 Positive test: negative control organisms *E.coli* ATCC®25922 (Tab 4) and carbapenemase positive (OXA-48) test organism *K.Pneumoniae* NCTC 13442(Tab 3); D: KPC Positive test: negative control organisms *E.coli* ATCC®25922 (Tab 4) and carbapenemase positive (KPC) test organism *K.Pneumoniae* NCTC 13438 (Tab 3); E: OXA-25 Positive test: negative control organisms *E.coli* ATCC®25922 (Tab 4) and carbapenemase positive (OXA-25) test organism *A.baumanii* NCTC 13302 (Tab 3); F: Acinetobacter negative test: negative control organism *E.coli* ATCC®25922 (Tab 4) and carbapenemase negative test organism *Acinetobacter* spp (Tab 3); G: Pseudomonas negative test: negative control organism *E.coli* ATCC25922 (Tab 4) and carbapenemase negative test organism *P.aeruginosa* ATCC®27853 (Tab 3); H: MBL positive test: negative control organism E.coli ATCC®25922 (Tab 4) and carbapenemase positive (MBL) test organism *P.aeruginosa* NCTC 13437 (disc 3).

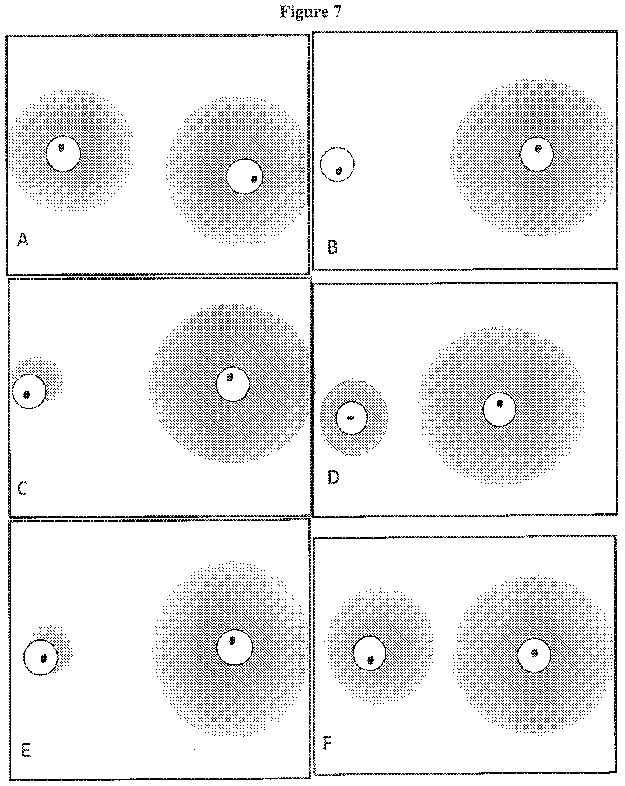

Figure 7 shows the results obtained in testing known carbapenem-hydrolysing β-lactamase bacteria in the single disc system of the present invention; A: Negative test: Carbapenemase negative test organism *E.coli*. (disc 4) and *.E.coli* ATCC®25922 (disc 5); B: MBL Positive test Carbapenemases inactivation test with no organism applied to negative control disc 4 and carbapenemase positive (MBL) organism *K. Pneumoniae* NCTC 13440 (disc 5); C: OXA-48 Positive test Carbapenemases inactivation test with no organism negative control (disc 4) and carbapenemase positive (OXA-48) organism *K. Pneumoniae* NCTC 13442 (disc 5); D: KPC Positive test Carbapenemases inactivation test with no organism negative control (disc 4) and carbapenemase positive (KPC) organism *K. Pneumoniae* NCTC 13438 (disc 5); E: OXA-25 Positive test Carbapenemases inactivation test with no organism negative control (disc 4) and carbapenemase positive (OXA-25) test organism *A. baumanii* NCTC 13302 (disc 5); F: Carbapenemases inactivation test with no organism negative control (disc 4) and carbapenemase negative organism *Acinetobacter* spp (disc 5).

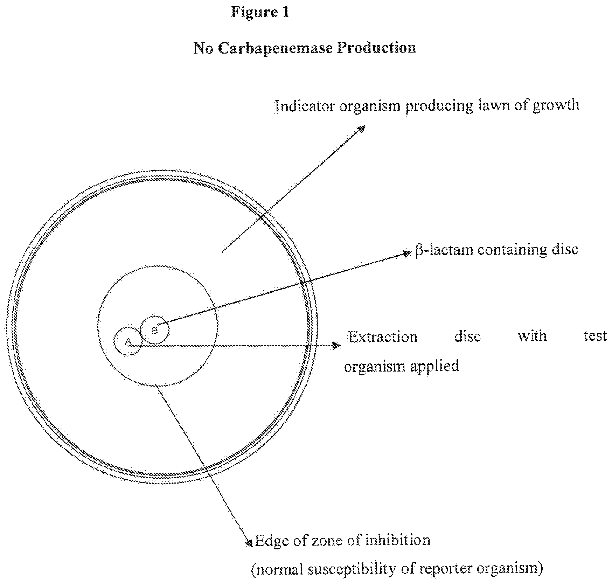

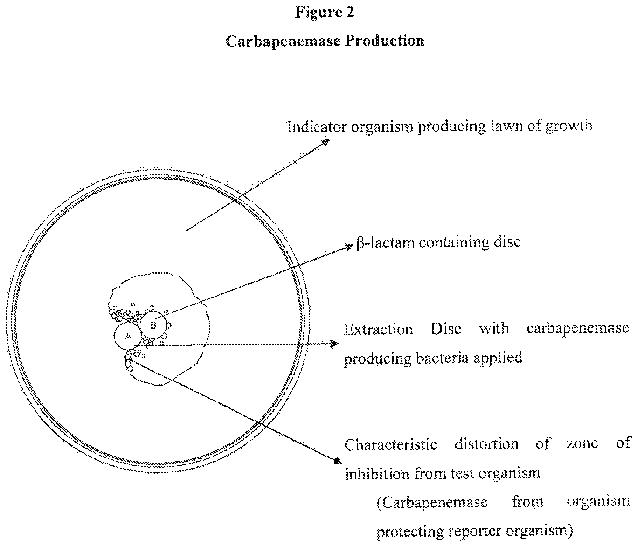

As illustrated in Figures 1 and 2, the method of the present invention may be performed using a dual disc assay system. Disc B is impregnated with a β-lactam containing antibiotic while extraction disc A is impregnated with an extended-spectrum β-lactamase inhibitor and/or an AmpC inhibitor, and ethylene glycol tetraacetic acid (EGTA). In use, a test sample thought to comprise carbapenem or penem hydrolysing β-lactamase is applied to the back of disc B, and discs And B are mounted on a culture plate which has been swabbed with a reporter organism. The discs are positioned on the plate to abut one another. Alternatively, the discs may be positioned to have a small gap there between. A suitable gap may be less than about 9 mm and preferably about 3 mm. After an incubation period, the plate is viewed to determine whether there test sample is positive or negative for a carbapenem or penem hydrolysing β-lactamase.

A positive test for a carbapenem or penem hydrolysing β-lactamase is shown in Figure 2 by a distorted zone of inhibition. A negative test for a carbapenem or penem hydrolysing β-lactamase is shown in Figure 1 by an undistorted zone of inhibition.

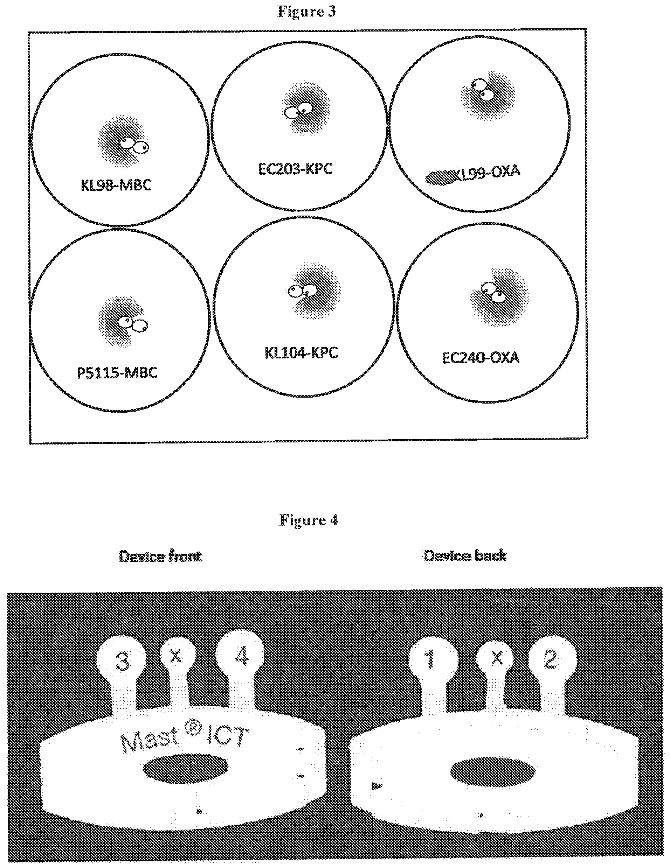

As illustrated in Figure 4, the method of the present invention may be performed using a triple disc assay system. The triple disc assay system comprises a central disc X, which is impregnated with a β-lactam containing antibiotic and two peripheral discs 3 and 4. Disc 3 is impregnated with an extended-spectrum β-lactamase inhibitor and/or an AmpC inhibitor, and ethylene glycol tetraacetic acid (EGTA). Disc 4 is a negative control disc and is also impregnated with extended-spectrum β-lactamase inhibitor and/or an AmpC inhibitor, and ethylene glycol tetraacetic acid (EGTA), and may also be impregnated with a negative control organism. A suitable negative control organism may be carbapenemase sensitive, such as *Escherichia coli* ATCC®25922.

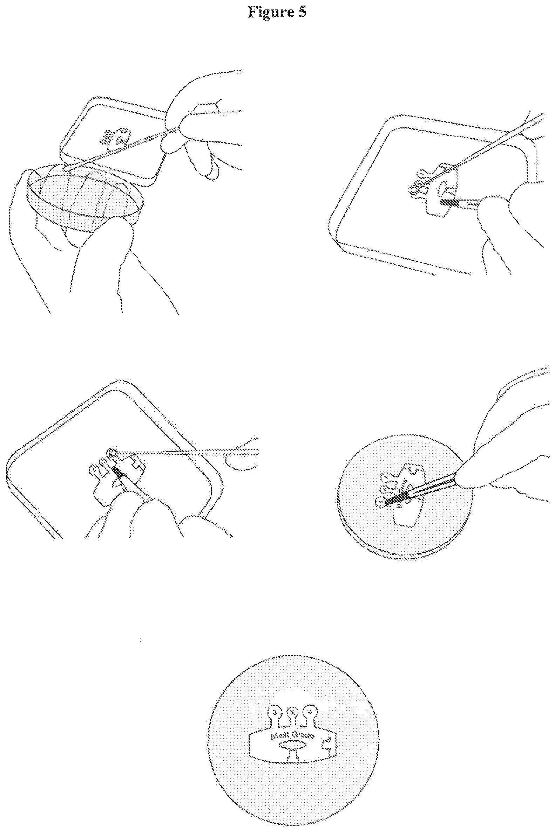

As shown in Figure 5, in use the triple disc assay system may be mounted on a culture plate which has been swabbed with a reporter organism. A test sample thought to comprise carbapenem or penem hydrolysing β-lactamase is then applied to the back of disc 3, while a negative control sample is applied to the back of disc 4 using sterile swabs. The triple disc assay system is then turned-over and mounted on the culture plate. The discs may then be pressed against the culture plate to ensure a good contact between the reporter organism and the impregnated discs. After an incubation period, the plate is viewed to determine whether there test sample is positive or negative for a carbapenem or penem hydrolysing β-lactamase.

A positive result is indicated by a dis-similar (distorted) zone of inhibition existing about the extraction tab compared to that of the negative control tab. This is shown by Figures 6B, 6C, 6D and 6H. A negative result is shown by figure 6A, 6E and 6F.

As illustrated in Figure 7, an alternative embodiment of the present invention provides a single assay disc system 5. In such a system disc 5 is impregnated with each of a β-lactam containing antibiotic, an extended-spectrum β-lactamase inhibitor and/or an AmpC inhibitor, and ethylene glycol tetraacetic acid (EGTA).

The method of the present invention is carried out by applying a test sample thought to comprise carbapenem or penem hydrolysing β-lactamase to the back of disc 5, which is then mounted on a culture plate which has been swabbed with a reporter organism, as depicted in Figure 5. A negative control disc which is also impregnated with a β-lactam containing antibiotic, an extended-spectrum β-lactamase inhibitor and/or an AmpC inhibitor, and ethylene glycol tetraacetic acid (EGTA), as well as a negative control organism, such as *Escherichia coli* ATCC®25922 may also be mounted on the culture plate.

Where a negative control disc has been mounted on the culture plate, a positive test for carbapenemase production is indicated by the presence of a clear reduction of zone of inhibition around the test disc in comparison to the negative control disc.

A negative test for carbapenemase production was indicated by the presence of minimal or no reduction in the zone of inhibition around the test disc in comparison to the negative control disc,

In any embodiment, an anerobic incubation step may be used when testing *P. Aeruginosa* samples. This may prevent overgrowth of an organism which may otherwise lead to misinterpretation of the resultant data.

### Examples

### Example 1

The Indirect Carbapenemase Test (ICT) was performed as previously described by Black J. et al (Journal of Clinical Microbiology, July 2005). 10µl of an EGTA solution to deliver 292µg/disc was impregnated onto blank discs available from MAST under product code BD0680W/C and left to dry. Once dry 10µl of a second a solution containing cloxacillin, Tazobactam, zinc sulphate, to deliver 100µg, 10µg and 121.8µg/ disc respectively was impregnated onto the same blank discs and left to dry for around 30 minutes. This disc with double impregnation formed the extraction disc.

For a test, a suspension of a carbapenem-susceptible *E. coli* strain (ATCC 25922) was prepared in saline (0.85% physiological equivalent) to a McFarland 0.5 turbidity standard and inoculated onto Mueller-Hinton agar plates to achieve a confluent lawn. Between 5 and 10 colonies of the test isolate were applied to the extraction disc by touching a few well-isolated colonies onto the disc using a 10µl loop in order to coat the extraction disc. The inoculated disc was placed with the bacterial inoculum (organism) side down on the lawn abutting a 10µg faropenem disc (available from MAST under catalogue number D71C- CATID). The plate was then incubated for 18-24 hours at 35°C±1 in ambient air.

Indentation of growth toward the CATID disk indicated a positive test for a carbapenem hydrolysing β-lactamase enzyme. This is shown schematically in Figure 1 (negative result) and Figure 2 (positive result).

The sensitivity of known carbapenem hydrolysing β-lactamase producing organisms was tested to confirm the efficacy of the test. The comprised KPC producing organisms, OXA producing organisms and MBL producing organisms. The results are shown in Figure 3 and show 100% sensitivity and specificity.

### Example 2

A method for determining whether a microorganism produces a carbapenem or penem hydrolysing β-lactamase according to an aspect of the present invention was tested using a triple disc carbapenemases inactivation (CAT ID) test.

A triple disc test system using a D74 designated disc obtained from Mast Group Limited, was used to perform in-house testing on a total of 120 carbapenemase positive and negative organisms. A positive test for carbapenemase production was indicated by a dis-similar (distorted) zone of inhibition existing about the extraction tab compared to that of the negative control tab.

**Table 1: in house test organism panel used in the triple CAT ID disc test**

| Test organisms | Carbapenemase positive organisms | Carbapenemase negative organisms |
|---|---|---|
| *Acinetobacter* spp | 7 | 15 |
| Enterobacteriaceae | 31 | 41 |
| *Pseudomonas* spp | 5 | 21 |

**Table 2: Results from in-house testing of the triple CAT ID disc test.**

| | Number of organisms |
|---|---|
| True Positive | 42 |
| True Negative | 69 |
| False Positive | 9 |
| False Negative | 0 |

As shown in Tables 1 and 2, nine organisms out of the 120 organisms tested gave false positive results. Of these 2 were de-repressed AmpC. The remaining seven false positive results are thought to be due to impurities and or contaminants affecting the purity of culture.

Thus the D74 device test gave 100% sensitivity, 88.5% specificity with a 100% NPV (negative predictive value) and 82.3% PPV (positive predictive value).

### Example 3

A method for determining whether a microorganism produces a carbapenem or penem hydrolysing β-lactamase according to an aspect of the present invention was tested using a single disc carbapenemases inactivation (CAT ID) test.

The tests were carried out as follows:
1. A pure, fresh culture of the reporter organism was used to prepare a suspension equivalent in density to a McFarland 0.5 opacity standard. When testing Enterobacteriaceae and *Acinetobacter* spp., *Escherichia coli* ATCC®25922 was used. When testing *Pseudomonas* spp, *Klebsiella pneumoniae* ATCC®700603 was used.
2. The suspension was then uniformly spread across the surface of a susceptibility test agar plate using a sterile swab.
3. The test disc was then transferred onto the sterile surface using sterile forceps.
4. A quantity of growth equivalent to approximately ¼ to ½ of a 10µl loopfuls of the organism to be tested was then applied to the test disc by rubbing the colonies onto the test disc covering as much of the area as possible.
5. The test disc was then placed on one side of the plate inoculated with the appropriate reporter organism using sterile forceps, insuring the face of the test disc inoculated with the test organism was facing down and was in contact with the media.
6. A negative control disc was then applied to the opposing side of the plate.
7. The organisms were incubated at 34°C-36°C aerobically for 16 to 20 hours when testing Enterobacteriaceae and *Acinetobacter* spp., or alternatively 34°C-36°C anaerobically for 16 to 20 hours when testing *Pseudomonas* spp.

The resultant sizes of the zones of inhibition were then studied to determine whether a microorganism producing a carbapenem or penem hydrolysing β-lactamase was present as shown in Figure 7A-7F.

A positive test for carbapenemase production was indicated by the presence of a clear reduction of zone of inhibition around the test disc in comparison to the negative control disc.

A negative test for carbapenemase production was indicated by the presence of minimal or no reduction in the zone of inhibition around the test disc in comparison to the negative control disc.

Thus, the present invention provides an a method for identifying bacteria which produce β-lactamases, particularly KPCs, MBLs and OXA-48 in Enterobacteriaceae, *Pseudomonas* spp & *Acinetobacter* spp. This involves the combination of the reagents used in preparing extraction disc and the Cat ID disc, in the previously stated quantities into a single disc.

The basis of the test method is the same as the two and three disc embodiments of the present invention. The permeabilizating agent (EGTA) exposes the carbapenemase enzymes present in the test organism which metabolizes any carbapenems present and in turn effect the zone of inhibition. The size of the overall zone of inhibition is affected. This is due to the carbapenem having to defuse though the permeabilizaed test organism before it can defuse into the surroundings media and effect the growth of the reporter organism forming the zone of inhibition.

## Claims

1. A method for determining whether a microorganism produces a carbapenem or penem hydrolysing β-lactamase, comprising:
providing a reagent composition comprising a β-lactam containing antibiotic, an extended-spectrum β-lactamase inhibitor and/or an AmpC inhibitor to which the carbapenem or penem hydrolysing β-lactamase is resistant, and a membrane permeabilizing agent which is ethylene glycol tetraacetic acid (EGTA) that permeabilizes the microorganism's outer membrane;
contacting the microorganism suspected of producing a β-lactamase with the reagent composition; and
culturing the microorganism with the reagent composition in a culture medium comprising a reporter microorganism wherein a positive test is determined by an increase in the growth of the reporter microorganism.

2. The method of Claim 1, wherein the carbapenem or penem-hydrolysing β-lactamase is a
metallo-β-lactamase, preferably produced by a *Pseudomonas* spp, Enterobacteriaceae or Acinetobacter spp.; or
a carbapenemase, preferably selected from Oxacillinases or a *K. pneumoniae* carbapenemase (KPC).

3. The method of Claim 1 or Claim 2, wherein the reagent composition is provided in at least two different discs wherein each disc comprises a carrier and the first disc has the β-lactam containing antibiotic impregnated in its carrier and the second disc has the extended-spectrum β-lactamase inhibitor and/or AmpC inhibitor and EGTA impregnated in its carrier.

4. The method of Claim 1 or Claim 2, wherein the reagent composition is provided in a single disc comprising the β-lactam containing antibiotic, the extended-spectrum β-lactamase inhibitor and/or AmpC inhibitor and the EGTA.

5. The method of Claim 3 or 4, wherein the disc comprising the extended-spectrum β-lactamase inhibitor and/or AmpC inhibitor and EGTA further comprises a divalent cation, preferably zinc ions, and optionally wherein the reagent composition further comprises a negative control disc.

6. The method of any preceding Claim, wherein β-lactam containing antibiotic is a carbapenem or penem, preferably selected from meropenem, imipenem, ertapenem, doripenem, faropenem, sulopenem, ritapenem, and preferably wherein the β-lactam containing antibiotic is faropenem.

7. The method of any preceding Claim, wherein the culture medium is a solid growth medium, optionally wherein the one or more discs are provided on the solid growth medium and wherein the test microorganism is applied to the disc having the EGTA, and further optionally wherein the microorganism is cultured with the reagent composition in a culture medium for from 16 to 24 hours at from 35 to 37°C.

8. The method of any of Claims 3 to 7, wherein the β-lactamase inhibitor is Cloxacillin present in an amount of from 67 to 150µg/ disc or Tazobactam from 6 to 15µg/disc or mixtures thereof.

9. The method of any of Claims 3 to 8, wherein the β-lactam containing antibiotic is present in an amount of from 1-25µg/disc, optionally wherein the EGTA is present in an amount of from 150 to 500µg/disc, and further optionally wherein zinc is present in the second disc at 75-150µg/disc.

10. An enzyme assay system for use in a method of determining whether a microorganism produces a carbapenem or penem-hydrolysing β-lactamase, the system comprising:
a β-lactam containing antibiotic;
an extended-spectrum β-lactamase inhibitor and/or an AmpC inhibitor to which the carbapenem or penem hydrolysing β-lactamase is resistant; and
a membrane permeabilizing agent which is ethylene glycol tetraacetic acid (EGTA) that permeabilizes the microorganism's outer membrane;
wherein
the system has one or more assay discs and the β-lactam containing antibiotic, extended-spectrum β-lactamase inhibitor and/or an AmpC inhibitor, and ethylene glycol tetraacetic acid (EGTA) are impregnated in the one or more discs.

11. The enzyme assay system of Claim 10, wherein the system further comprises a negative control disc, and optionally wherein the β-lactam containing antibiotic, extended-spectrum β-lactamase inhibitor and/or an AmpC inhibitor, and ethylene glycol tetraacetic acid (EGTA) are all impregnated in a single assay disc.

12. The enzyme assay system of any of Claims 10 to 11, wherein the system comprises a first assay disc comprising a β-lactam containing antibiotic, and a second assay disc comprising an extended-spectrum β-lactamase inhibitor and/or an AmpC inhibitor, and ethylene glycol tetraacetic acid (EGTA).

13. The enzyme assay system of any of Claims 10 to 12, wherein the system comprises a first assay disc comprising a β-lactam containing antibiotic, a second assay disc comprising an extended-spectrum β-lactamase inhibitor and/or an AmpC inhibitor, and ethylene glycol tetraacetic acid (EGTA), and a third assay disc which is a negative control disc.

14. The enzyme assay system of Claim 13, wherein each of the discs is mounted on a disc support which maintains the three discs at an optimised position relative to each other, such that the first assay disc comprising a β-lactam containing antibiotic is centrally located between the second assay disc comprising an extended-spectrum β-lactamase inhibitor and/or an AmpC inhibitor, and ethylene glycol tetraacetic acid (EGTA), and the negative control disc.

15. A kit for determining whether a microorganism produces a carbapenem-hydrolysing β-lactamase, comprising an enzyme assay system according to any of Claims 10 to Claim 14.

## Patentansprüche

1. Verfahren für die Bestimmung, ob ein Mikroorganismus eine Carbapenem- oder Penem-hydrolysierende β-Lactamase erzeugt, das Folgendes umfasst:
Bereitstellen einer Reagenzzusammensetzung, die Folgendes umfasst: ein β-Lactam-enthaltendes Antibiotikum, einen Breitspektrum-ß-Lactamase-Inhibitor und/oder einen AmpC-Inhibitor, gegen den die Carbapenem- oder Penem-hydrolysierende β-Lactamase resistent ist, und ein Membran-durchdringendes Mittel, das Ethylenglycoltetraessigsäure (EGTA) ist, die die äußere Membran des Mikroorganismus durchdringt;
Inkontaktbringen des Organismus, von dem vermutet wird, dass er eine β-Lactamase erzeugt, mit der Reagenzzusammensetzung; und
Kultivieren des Mikroorganismus mit der Reagenzzusammensetzung in einem Kulturmedium, das einen Reporter-Mikroorganismus umfasst, wobei ein positiver Test durch einen Anstieg des Wachstums des Reporter-Mikroorganismus bestimmt wird.

2. Verfahren nach Anspruch 1, wobei die Carbapenem- oder Penem-hydrolysierende β-Lactamase Folgendes ist:
eine Metallo-ß-Lactamase, die bevorzugt von einer *Pseudomonas* spp., Enterobacteriaceae oder Acinetobacter spp. erzeugt wird; oder
eine Carbapenemase, die bevorzugt aus Oxacillinasen oder einer *K. pneumoniae* Carbapenemase (KPC) ausgewählt ist.

3. Verfahren nach Anspruch 1 oder Anspruch 2, wobei die Reagenzzusammensetzung in mindestens zwei verschiedenen Scheibchen bereitgestellt wird, wobei jedes Scheibchen einen Träger umfasst und das erste Scheibchen das β-Lactam-enthaltende Antibiotikum in seinem Träger imprägnierend aufweist und das zweite Scheibchen den Breitspektrum-β-Lactamase-Inhibitor und/oder AmpC-Inhibitor und die EGTA in seinem Träger imprägnierend aufweist.

4. Verfahren nach Anspruch 1 oder Anspruch 2, wobei die Reagenzzusammensetzung in einem einzigen Scheibchen bereitgestellt wird, das das β-Lactam-enthaltende Antibiotikum, den Breitspektrum-β-Lactamase-Inhibitor und/oder AmpC-Inhibitor und die EGTA umfasst.

5. Verfahren nach Anspruch 3 oder 4, wobei das Scheibchen, das den Breitspektrum-ß-Lactamase-Inhibitor und/oder AmpC-Inhibitor und die EGTA umfasst, weiter ein zweiwertiges Kation umfasst, bevorzugt Zinkionen, und optional wobei die Reagenzzusammensetzung weiter ein Scheibchen der negativen Kontrolle umfasst.

6. Verfahren nach einem vorstehenden Anspruch, wobei das β-Lactam-enthaltende Antibiotikum ein Carbapenem oder Penem ist, das bevorzugt aus Meropenem, Imipenem, Ertapenem, Doripenem, Faropenem, Sulopenem, Ritapenem ausgewählt ist, und bevorzugt wobei das β-Lactam-enthaltende Antibiotikum Faropenem ist.

7. Verfahren nach einem vorstehenden Anspruch, wobei das Kulturmedium ein festes Wachstumsmedium ist, optional wobei das eine oder die mehreren Scheibchen auf dem festen Wachstumsmedium bereitgestellt sind und wobei der Testmikroorganismus auf das Scheibchen aufgetragen wird, das die EGTA aufweist, und weiter optional wobei der Mikroorganismus mit der Reagenzzusammensetzung in einem Kulturmedium für 16 bis 24 Stunden bei 35 bis 37 °C kultiviert wird.

8. Verfahren nach einem der Ansprüche 3 bis 7, wobei der β-Lactamase-Inhibitor Cloxacillin, das in einer Menge von 67 bis 150 µg/Scheibchen vorliegt, oder Tazobactam von 6 bis 15 µg/Scheibchen oder Mischungen davon ist.

9. Verfahren nach einem der Ansprüche 3 bis 8, wobei das β-Lactam-enthaltende Antibiotikum in einer Menge von 1-25 µg/Scheibchen vorliegt, optional wobei die EGTA in einer Menge von 150 bis 500 µg/Scheibchen vorliegt und weiter optional wobei Zink in dem zweiten Scheibchen zu 75-150 µg/Scheibchen vorliegt.

10. Enzym-Assay-System für die Verwendung in einem Verfahren zur Bestimmung, ob ein Mikroorganismus eine Carbapenem- oder Penem-hydrolysierende β-Lactamase erzeugt, wobei das System Folgendes umfasst:
ein β-Lactam-enthaltendes Antibiotikum;
einen Breitspektrum-β-Lactamase-Inhibitor und/oder einen AmpC-Inhibitor, gegen den die Carbapenem- oder Penem-hydrolysierende β-Lactamase resistent ist; und
ein Membran-durchdringendes Mittel, das Ethylenglycoltetraessigsäure (EGTA) ist, die die äußere Membran des Mikroorganismus durchdringt;
wobei
das System ein oder mehrere Assay-Scheibchen aufweist und das β-Lactam-enthaltende Antibiotikum, der Breitspektrum-β-Lactamase-Inhibitor und/oder ein AmpC-Inhibitor und Ethylenglycoltetraessigsäure (EGTA) in dem einen oder den mehreren Scheibchen imprägnierend vorliegen.

11. Enzym-Assay-System nach Anspruch 10, wobei das System weiter ein Scheibchen der negativen Kontrolle umfasst und optional wobei das β-Lactam-enthaltende Antibiotikum, der Breitspektrum-β-Lactamase-Inhibitor und/oder ein AmpC-Inhibitor und Ethylenglycoltetraessigsäure (EGTA) alle in einem einzigen Assay-Scheibchen imprägnierend vorliegen.

12. Enzym-Assay-System nach einem der Ansprüche 10 bis 11, wobei das System Folgendes umfasst: ein erstes Assay-Scheibchen, das ein β-Lactam-enthaltendes Antibiotikum umfasst, und ein zweites Assay-Scheibchen, das einen Breitspektrum-β-Lactamase-Inhibitor und/oder einen AmpC-Inhibitor und Ethylenglycoltetraessigsäure (EGTA) umfasst.

13. Enzym-Assay-System nach einem der Ansprüche 10 bis 12, wobei das System Folgendes umfasst: ein erstes Assay-Scheibchen, das ein β-Lactam-enthaltendes Antibiotikum umfasst, ein zweites Assay-Scheibchen, das einen Breitspektrum-β-Lactamase-Inhibitor und/oder einen AmpC-Inhibitor und Ethylenglycoltetraessigsäure (EGTA) umfasst, und ein drittes Assay-Scheibchen, das ein Scheibchen der negativen Kontrolle ist.

14. Enzym-Assay-System nach Anspruch 13, wobei jedes der Scheibchen auf einem Scheibchenträger angebracht ist, der die drei Scheibchen an einer optimierten Position in Bezug aufeinander hält, sodass sich das erste Assay-Scheibchen, das ein β-Lactam-enthaltendes Antibiotikum umfasst, mittig zwischen dem zweiten Assay-Scheibchen, das einen Breitspektrum-β-Lactamase-Inhibitor und/oder einen AmpC-Inhibitor und Ethylenglycoltetraessigsäure (EGTA) umfasst, und dem Scheibchen der negativen Kontrolle befindet.

15. Kit für die Bestimmung, ob ein Mikroorganismus eine Carbapenemhydrolysierende β-Lactamase erzeugt, der ein Enzym-Assay-System nach einem von Anspruch 10 bis Anspruch 14 umfasst.

## Revendications

1. Méthode de détermination si un microorganisme produit ou non une β-lactamase hydrolysant les carbapénèmes ou les pénèmes, comprenant :
la fourniture d'une composition de réactif comprenant un antibiotique contenant du β-lactame, un inhibiteur de β-lactamase à spectre étendu et/ou un inhibiteur d'AMPc vis-à-vis desquels la β-lactamase hydrolysant les carbapénèmes ou les pénèmes est résistante, et un agent de perméabilisation membranaire qui est l'acide éthylène glycol tétraacétique (EGTA) qui perméabilise la membrane externe du microorganisme ;
la mise en contact du microorganisme suspecté de produire une β-lactamase avec la composition de réactif ; et
la culture du microorganisme avec la composition de réactif dans un milieu de culture comprenant un microorganisme rapporteur, où un test positif est déterminé par une augmentation de la croissance du microorganisme rapporteur.

2. Méthode selon la revendication 1, dans laquelle la β-lactamase hydrolysant les carbapénèmes ou les pénèmes est
une métallo-β-lactamase, produite de préférence par *Pseudomonas* spp., *Enterobacteriaceae* ou *Acinetobacter* spp. ; ou
une carbapénémase, choisie de préférence parmi les oxacillinases ou une carbapénémase de *K. pneumoniae* (KPC).

3. Méthode selon la revendication 1 ou la revendication 2, dans laquelle la composition de réactif est fournie dans au moins deux disques différents, où chaque disque comprend un véhicule et le premier disque possède l'antibiotique contenant du β-lactame imprégné dans son véhicule et le deuxième disque possède l'inhibiteur de β-lactamase à spectre étendu et/ou l'inhibiteur d'AMPc et de l'EGTA imprégnés dans son véhicule.

4. Méthode selon la revendication 1 ou la revendication 2, dans laquelle la composition de réactif est fournie dans un disque unique, comprenant l'antibiotique contenant du β-lactame, l'inhibiteur de β-lactamase à spectre étendu et/ou l'inhibiteur d'AMPc et l'EGTA.

5. Méthode selon la revendication 3 ou 4, dans laquelle le disque comprenant l'inhibiteur de β-lactamase à spectre étendu et/ou l'inhibiteur d'AMPc et l'EGTA, comprend en outre un cation divalent, préférablement des ions zinc, et éventuellement où la composition de réactif comprend en outre un disque de témoin négatif.

6. Méthode selon l'une quelconque des revendications précédentes, dans laquelle l'antibiotique contenant du β-lactame est un carbapénème ou un pénème, choisi de préférence parmi le méropénème, l'imipénème, l'ertapénème, le doripénème, le faropénème, le sulopénème, le ritapénème, et préférablement où l'antibiotique contenant du β-lactame est le faropénème.

7. Méthode selon l'une quelconque des revendications précédentes, dans laquelle le milieu de culture est un milieu de culture solide, éventuellement où les un ou plusieurs disques sont fournis sur le milieu de culture solide et où le microorganisme à tester est appliqué sur le disque possédant l'EGTA, et en outre éventuellement où le microorganisme est cultivé avec la composition de réactif dans un milieu de culture pendant de 16 à 24 heures à de 35 à 37°C.

8. Méthode selon l'une quelconque des revendications 3 à 7, dans laquelle l'inhibiteur de β-lactamase est constitué de cloxacilline présente selon une quantité allant de 67 à 150 µg/disque ou de tazobactam selon de 6 à 15 µg/disque ou de mélanges de ceux-ci.

9. Méthode selon l'une quelconque des revendications 3 à 8, dans laquelle l'antibiotique contenant du β-lactame est présent selon une quantité de 1-25 µg/disque, éventuellement où l'EGTA est présent selon une quantité allant de 150 à 500 µg/disque, et en outre éventuellement où du zinc est présent dans le deuxième disque à 75-150 µg/disque.

10. Système de dosage enzymatique destiné à une utilisation dans une méthode de détermination si un microorganisme produit ou non une β-lactamase hydrolysant les carbapénèmes ou les pénèmes, le système comprenant :
un antibiotique contenant du β-lactame ;
un inhibiteur de β-lactamase à spectre étendu et/ou un inhibiteur d'AMPc vis-à-vis desquels la β-lactamase hydrolysant les carbapénèmes ou les pénèmes est résistante ; et
un agent de perméabilisation membranaire qui est l'acide éthylène glycol tétraacétique (EGTA) qui perméabilise la membrane externe du microorganisme ;
où
le système possède un ou plusieurs disques de dosage et l'antibiotique contenant du β-lactame, l'inhibiteur de β-lactamase à spectre étendu et/ou un inhibiteur d'AMPc et l'acide éthylène glycol tétraacétique (EGTA), sont imprégnés dans les un ou plusieurs disques.

11. Système de dosage enzymatique selon la revendication 10, où le système comprend en outre un disque de témoin négatif, et éventuellement où l'antibiotique contenant du β-lactame, l'inhibiteur de β-lactamase à spectre étendu et/ou un inhibiteur d'AMPc et l'acide éthylène glycol tétraacétique (EGTA), sont tous imprégnés dans un seul disque de dosage.

12. Système de dosage enzymatique selon l'une quelconque des revendications 10 à 11, où le système comprend un premier disque de dosage comprenant un antibiotique contenant du β-lactame, et un deuxième disque de dosage comprenant un inhibiteur de β-lactamase à spectre étendu et/ou un inhibiteur d'AMPc, et l'acide éthylène glycol tétraacétique (EGTA).

13. Système de dosage enzymatique selon l'une quelconque des revendications 10 à 12, où le système comprend un premier disque de dosage comprenant un antibiotique contenant du β-lactame, un deuxième disque de dosage comprenant un inhibiteur de β-lactamase à spectre étendu et/ou un inhibiteur d'AMPc, et l'acide éthylène glycol tétraacétique (EGTA), et un troisième disque de dosage qui est un disque de témoin négatif.

14. Système de dosage enzymatique selon la revendication 13, où chacun des disques est monté sur un support de disques qui maintient les trois disques au niveau d'une position optimisée les uns par rapport aux autres, de sorte que le premier disque de dosage comprenant un antibiotique contenant du β-lactame est situé centralement entre le deuxième disque de dosage comprenant un inhibiteur de β-lactamase à spectre étendu et/ou un inhibiteur d'AMPc, et l'acide éthylène glycol tétraacétique (EGTA), et le disque de témoin négatif.

15. Kit destiné à la détermination si un microorganisme produit ou non une β-lactamase hydrolysant les carbapénèmes, comprenant un système de dosage enzymatique selon l'une quelconque parmi la revendication 10 à la revendication 14.
